Europäisches Patentamt

European Patent Office

Office européen des brevets

(19)

(11) Veröffentlichungsnummer : **0 357 163 B1**

(12)                    # EUROPÄISCHE PATENTSCHRIFT

(45) Veröffentlichungstag der Patentschrift :
03.06.92 Patentblatt 92/23

(51) Int. Cl.⁵ : **A61K 49/00, A61K 49/04**

(21) Anmeldenummer : **89250016.6**

(22) Anmeldetag : **18.08.89**

(54) Mittel bestehend aus Cavitate oder Clathrate bildenden Wirt/Gast-Komplexen als Kontrastmittel.

(30) Priorität : **23.08.88 DE 3828905**

(43) Veröffentlichungstag der Anmeldung :
**07.03.90 Patentblatt 90/10**

(45) Bekanntmachung des Hinweises auf die
Patenterteilung :
**03.06.92 Patentblatt 92/23**

(84) Benannte Vertragsstaaten :
**AT BE CH DE ES FR GB GR IT LI LU NL SE**

(56) Entgegenhaltungen :
EP-A- 0 224 934
WO-A-80/02365
DE-A- 3 637 926
PATENT ABSTRACTS OF JAPAN, Band 5, Nr.
160 (C-75)[832], 15. Oktober 1981; & JP-A-56 92
221 (ZERIA SHINYAKU KOGYO K.K.)25-
07-1981
F. Cramer, Angew. Chem., 68 (1956) 115/20.
D.D. MacNicol, Inclusion Compounds 2, Academic Press, London, (1984), 1/45.

(56) Entgegenhaltungen :
F. Vögtle et al., Angew. Chem., 97 (1985)
721/36.
E. C. Makin, Clathration, 6, 179/89.
S.G. Frank. Journal of Pharmaceutical Sciences, 64 (1975) 1585/604.
F. Cramer, Angew. Chem., 64 (1952) 437/47.
H. Remy, Lehrbuch der Anorganischen Chemie Bd. I, Akademische Verlagsgesellschaft
Geest & Portig K.-G., Leipzig (1965)263ff.

(73) Patentinhaber : SCHERING
AKTIENGESELLSCHAFT Berlin und
Bergkamen
Müllerstrasse 170/178 Postfach 65 03 11
W-1000 Berlin 65 (DE)

(72) Erfinder : Albayrak, Celal, Dr.
Svenemünder Strasse 92
W-1000 Berlin 65 (DE)
Erfinder : Rössling, Georg, Dr.
Oranienburger Chaussee 60 c
W-1000 Berlin 28 (DE)
Erfinder : Tack, Johannes, Dr.
Tharsanderweg 42
W-1000 Berlin 20 (DE)

EP 0 357 163 B1

## Beschreibung

Die Erfindung betrifft ein Mittel bestehend aus Cavitate- oder Clathrate Wirt/Gast-Komplexen, deren Wirtmoleküle sich in einem flüssigen Vehikel unter freisetzung des Gastes auflösen als kontrast mittel bei Ultraschull-, Röntgen- oder NMR-UNtersuchungen. In der Literatur wird die Herstellung von stöchiometrischen Wirt/Gast-Komplexen bestehend aus Wirtmolekülen, im wesentlichen organischen Oniumverbindungen und Gasen bzw. Gasbildnern als Gastmoleküle beschrieben (Angew. Chem. 97 (1985) 721). Eine Verwendung der Wirt/Gast-Komplexe als Konstrastmittel wird nicht beschrieben.

Der Erfindung liegt die Aufgabe zugrunde, für die Ultraschall-, Röntgen- oder NMR-Untersuchungen ein Mittel zur Verfügung zu stellen, das als Transportmedium für Kontrastmittel verwendet werden kann. Insbesondere sollen durch die Erfindung Wirt/-Gast-Komplexe zur Verfügung gestellt werden, die größtmögliche Gastvolumina in einer minimalen Wirtmasse speichern.

Überraschenderweise wurde gefunden, daß die genannten Cavitate- oder Clathrate-Wirt/-Gast - Komplexe ein Transportmittel bilden, die sich vollständig zersetzen und so gewählt werden können, daß sie keinerlei toxische Einflüsse auf die biologische Substanz ausüben, in der die Untersuchung durchgeführt werden soll.

Mit Vorteil kann das verwendete Mittel zur Ultraschalluntersuchung als Wirtmoleküle.

Wasser, Harnstoff und dessen Derivate, Thioharnstoff und dessen Derivate, Phenol und substituierte Phenole, Dihydroxybenzole und dessen Derivate, Hydrochinon und substituierte Hydrochinone, Salicylsäure und deren Derivate, Tri-o-Thymotid und dessen Derivate, Ascorbinsäure, Flavine und deren Derivate, Flavanole und deren Derivate, Cyclophane und deren Derivate, Guayacamin, Naphtohydrochinone und deren Derivate, Cyclodextrin und dessen Derivate, insbesondere Dimethyl-1-cyclodextrin, Methyl-$\beta$-cyclodextrin, Hydroxypro-pyl-$\beta$-cyclodextrin, Chromane und deren Derivate, insbesondere 4-p-Hydroxyphenyl-2,2,4-trimethylchroman, 4-p-Hydroxyphenyl-2,2,4-trimethylthiochroman, 4-p-Hydroxyphenyl-2,2,4,7-tetramethylthiochroman, 4-p-Hydroxyphenyl-2,2,4-trimethylselenchroman, Hexahostverbindungen, insbesondere Hexakis(phenylthio)benzol und dessen Derivate, Cyclotriveratrylen und dessen Derivate, 1,1'-Binaphtyl-2,2'-dicarbonsäure und deren Derivate, Oniumverbindungen und deren Derivate, Acetylsalicylsäure, Di-, Tri- und Tetrasalicylide, 9,9'-Spirobifluoren-2,2'-dicarboxylacid, Choleinsäuren, 4-4'Dinitrodiphenyl, Bis-(N,N'-alkylenbenzidin), Bis-(N,N'-tetramethylen-benzidin), Desoxycholsäure, Monoaminonickel(II)-cyanide, Tetra(4-methylpyridin)-Nickel(II)-dithiocyanate und deren Derivate, Hexamethylisocyanidoferronchloride, 2-phenyl-3-p(2,2,4-trimethylchroman-4-yl)-phenylquinazolin-4, Cyclotriphosphazone, Tris-1,2-phenyldioxycyclotriphosphazone

als Gastmoleküle:
Edelgase und Edelgasverbindungen, Schwefelhalogenide, Stickstoff und Stickstoffoxide, Kohlenstoffoxide, Wasserstoff und Wasserstoffoxide, Schwefeloxide, Phosphorwasserstoffe, Halogenwasserstoffe, Uranhalogenide und Sauerstoff sowie Kohlenwasserstoffe und deren Derivate, Epoxide, Ether und halogenierte Kohlernwasserstoffe enthalten.

Mit besonderem Vorteil kann das verwendete Mittel zur Ultraschalluntersuchung als Gastmoleküle Helium, Neon, Argon, Krypton, Xenon, Radon, Schwefelhexafluorid, Wasserstoff, Wasserstoffperoxid, Stickstoffmonoxid, Kohlenmonoxid, Kohlendioxid, Jodwasserstoff, Xenondifluorid, Xenontetrafluorid, Xenonhexafluorid, Xenondioxid, Schwefeldioxid, Schwefeltrioxid, Arsenwasserstoff, Phosphorwasserstoff, Deuterium, Uranhexafluorid, Methan, Ethan, Propan, Cyclopropan, Butan, Pentan, Ethylenoxid und Methylbromid enthalten.

Die kristallinen Komplexe können in ihrer Korngröße, insbesondere durch die Kristallisationsbedingungen sowie auch durch mechanische Verfahren der Partikelzerkleinerung (Luftstrahlvermahlung) beeinflußt werden.

Die kristallinen Komplexe können mit hydrophilen, lipophilen oder amphiphilen Hilfsstoffen überzogen werden.

Als Vehikel zur Applikation der Komplexe eignen sich sterile wässrige Systeme mit Zusätzen zur Einstellung von Viskosität, Oberflächenspannung, pH-Wert und osmotischem Druck, in denen die Komplexe vorzugsweise vor der Anwendung gelöst, aber auch suspendiert und ggf. emulgiert werden.

Die Wirt/Gast-Komplexe werden in ein wässriges Vehikel eingebracht. Durch die Auflösung der Wirtmoleküle werden die Komplexe unter Freisetzung der Gasblasen in das Vehikel zerstört. Die im Vehikel gelösten Wirtmoleküle haben keine komplexierenden Eigenschaften mehr. Die Geschwindigkeit der Gasfreisetzung, die Größe und Lebensdauer der Gasblasen können durch die Art des eingeschlossenen Gases oder des Gasbildners, durch die Art des Wirtmoleküls und von der Oberfläche bzw. Korngröße, in Abhängigkeit von Viskosität, Oberflächenspannung des Vehikels in weiten Grenzen eingestellt werden.

Es gelingt somit überraschenderweise auf einem sehr einfachen Wege injizierbare, gashaltige, pharmazeutische Zubereitungen mit ausgeprägten, echogenen Eigenschaften zu erhalten.

Insbesondere gelingt es, die für die in vivo Kontrastierung z. B. des linken Ventrikels beim Menschen notwendige Gasmenge von ca. 150 µl durch sehr geringe Wirkstoffmengen im Bereich von 2 - 10 mg/Appl. bereitzustellen, wie folgende Zusammenstellung zeigt:

```
Hydrochinon/N₂            3:1 Komplex  1 mg   70 µl
Hydrochinon/Xe           3:1    "      1 mg   53 µl
Dianin/SF₆               3:1    "      1 mg   26 µl
Dianin/Argon             2:1    "      1 mg   26 µl
Tri-o-Thymotide/Methan   2:1    "      1 mg   23 µl
Tri-o-Thymotide CH₃Br    2:1    "      1 mg   21 µl
Dianin/N2                               1 mg  103 µl
```

4-(4-Hydroxyphenyl)-2,2,4-trimethyl-chroman) wird als Dianin-Verbindung bezeichnet und hergestellt gem. Lit.-Stellen J. Russ Phys. Chem. Soc. 46, 1310 (1914) und Chem. Zentr. 1915, I, 1063.

Damit gelingt es, ein Kontrastmittel für die Ultraschall-Diagnostik bereitzustellen, das in der Lage ist, nach intravenöser Applikation das Blut und dessen Strömungsverhältnisse auf der rechten und nach Passage des Lungenkapillarbettes auf der linken Seite des Herzens für Ultraschall sichtbar zu machen. Darüberhinaus soll es auch die Darstellung der Durchblutung anderer Organe wie Myocard, Leber, Milz und Niere gestatten. Ebenso ist die Anwendung zur Darstellung der ableitenden Harnwege und des Magen-Darm-Traktes, der Gelenke, der Stirnhöhle und der Augen möglich.

Insbesondere bei der Verwendung von Gasmolekülen (z.B. Xenon), die in der Lage sind, die Blut/Hirn-Schranke zu überwinden, ist die Darstellung des Gehirns und seiner physiologischen und pathologischen Strukturen durch Ultraschall möglich.

Enthält das erfindungsgemäße Mittel z. B. Xenon ist die Verwendung dieser Wirt/Gast-Komplexe als Röntgenkontrastmittel möglich. Bei Verwendung stabiler Radikale (z.B. Sauerstoff-, Nitroxyl-) können die erfindungsgemäßen Mittel auch als NMR-Kontrastmittel eingesetzt werden.

Im folgenden wird die Erfindung anhand von Beispielen erläutert:

1. Tri-o-Thymotide/Methylbromid

Tri-o-Thymotide (25 g) wurde in 2.2.4-Trimethylpentan (50 ml) bei 100°C gelöst und die heiße Lösung in den Hochdruckautoklaven eingebracht. In den Autoklaven wurde soviel Methylbromid eingefüllt, bis ein Druck von 200 bar erreicht wurde. Der Hochdruckautoklav wurde 2 h lang auf 110°C temperiert und anschließend die Lösung innerhalb von 5 Tagen auf Raumtemperatur abgekühlt. Die Kristalle wurden abfiltriert und 3 mal mit kaltem 2.2.4-Trimethylpentan gewaschen; anschließend wurden die Kristalle im Trockenschrank bei 50°C getrocknet.

2. Dianin-Verbindung (4-p-Hydroxyphenyl-2.2.4-trimethylchroman)/Ethylenoxid

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Ethylenoxid von 300 bar beaufschlagt. Der Hochdruckautoklav wurde 2 h lang auf 140°C temperiert und anschließend die Lösung innerhalb von 8 Tagen auf Raumtemperatur abgekühlt. Die Kristalle wurden abfiltriert und 4 mal mit kaltem 1-Decanol (5 ml) gewaschen; anschließend wurden die Kristalle im Trockenschrank bei 100°C getrocknet.

3. Dianin-Verbindung (4-p-Hydroxyphenyl-2.2.4-trimethylchroman)/Schwefelhexafluorid

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Schwefelhexafluorid von 300 bar beaufschlagt. Der Hochdruckautoklav wurde 2 h lang auf 140° C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.

4. Dianin-Verbindung (4-p-Hydroxyphenyl-2.2.4-trimethylchroman)/Ethan

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Ethan von 300 bar beaufschlagt. Der Hochdruckautoklav wurde 2 h lang auf 140°C temperiert.

Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.

5. Dianin-Verbindung (4-p-Hydroxyphenyl-2.2.4-trimethylchroman)/Propan

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Propan von 300 bar beaufschlagt. Der Hochdruckautoklav wurde 2 h lang auf 140 °C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.

6. Dianin-Verbindung (4-p-Hydroxyphenyl-2.2.4-trimethylchroman)/Kohlendioxid

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Kohlendioxid von 300 bar beaufschlagt. Der Hochdruckautoklav wurde 2 h lang auf 140 ° C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.

7. Dianin-Verbindung (4-p-Hydroxyphenyl-2.2.4-trimethylchroman)/Cyclopropan

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Cyclopropan von 300 bar beaufschlagt. Der Hochdruckautoklav wurde 2 h lang auf 140° C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.

8. Dianin-Verbindung (4-p-Hydroxyphenyl-2.2.4-trimethylchroman)/Methan

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Methan von 300 bar beaufschlagt. Der Hochdruckautoklav wurde 2 h lang auf 140°C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.

9. Dianin-Verbindung (4-p-Hydroxyphenyl-2.2.4-trimethylchroman)/Stickstoff

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Stickstoff von 300 bar beaufschlagt. Der Hochdruckautoklav wurde 2 h lang auf 140 ° C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.
Schmelzpunkt: 162,88°C

10. Dianin-Verbindung (4-p-Hydroxyphenyl-2.2.4-trimethylchroman)/Xenon

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Xenon von 300 bar beaufschlagt. Der Hochdruckautoklav wurde 2 h lang auf 140 ° C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100 °C getrocknet.

11. Dianin-Verbindung (4-p-Hydroxyphenyl-2.2.4-trimethylchroman)/Argon

Dianin-Verbindung (25 g) wurde in 1-Decanol (35 g) bei 125°C gelöst. Die heiße Lösung wurde in den Hochdruckautoklaven gebracht. Die Lösung wurde mit komprimiertem Argon von 300 bar beaufschlagt. Der Hochdruckautoklav wurde 2 h lang auf 140°C temperiert. Anschließend wurde die Lösung innerhalb von 8 Tagen auf Raumtemperatur abgekühlt. Die Kristalle wurden abfiltriert und 4 mal mit kaltem 1-Decanol (5 ml) gewaschen. Anschließend wurden die Kristalle im Trockenschrank bei 100°C getrocknet.
Schmelzpunkt: 160,84°C

12. Hydrochinon/Methan

Hydrochinon (30 g) wurde in n-Propanol (70 ml) bei 70° C gelöst. Die heiße Lösung wurde in den Hochdruckautoklaven gebracht. Die Lösung wurde mit komprimiertem Methan von 300 bar beaufschlagt. Der Hochdruckautoklav wurde 2 h lang auf 80°C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

13. Hydrochinon/Schwefelhexafluorid

Hydrochinon (30 g) wurde in n-Propanol (70 ml) bei 70° C gelöst. Die heiße Lösung wurde in den Hochdruckautoklaven gebracht. Die Lösung wurde mit komprimiertem Schwefelhexafluorid von 300 bar beaufschlagt. Der Hochdruckautoklav wurde 2 h lang auf 80°C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

14. Hydrochinon/Propan

Hydrochinon (30 g) wurde in n-Propanol (70 ml) bei 70° C gelöst. Die heiße Lösung wurde in den Hochdruckautoklaven gebracht. Die Lösung wurde mit komprimiertem Propan von 300 bar beaufschlagt. Der Hochdruckautoklav wurde 2 h lang auf 80°C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

15. Hydrochinon/Ethan

Hydrochinon (30 g) wurde in n-Propanol (70 ml) bei 70° C gelöst. Die heiße Lösung wurde in den Hochdruckautoklaven gebracht. Die Lösung wurde mit komprimiertem Ethan von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70 °C getrocknet.

16. Hydrochinon/Kohlendioxid

Hydrochinon (30 g) wurde in n-Propanol (70 ml) bei 70° C gelöst. Die heiße Lösung wurde in den Hochdruckautoklaven gebracht. Die Lösung wurde mit komprimiertem Kohlendioxid von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

17. Hydrochinon/Ethylenoxid

Hydrochinon (30 g) wurde in n-Propanol (70 ml) bei 70° C gelöst. Die heiße Lösung wurde in den Hochdruckautoklaven gebracht. Die Lösung wurde mit komprimiertem Ethylenoxid von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

### 18. Hydrochinon/Cyclopropan

Hydrochinon (30 g) wurde in n-Propanol (70 ml) bei 70° C gelöst. Die heiße Lösung wurde in den Hochdruckautoklaven gebracht. Die Lösung wurde mit komprimiertem Cyclopropan von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

### 19. Hydrochinon/Stickstoff

Hydrochinon (30 g) wurde in n-Propanol (70 ml) bei 70° C gelöst. Die heiße Lösung wurde in den Hochdruckautoklaven gebracht. Die Lösung wurde mit komprimiertem Stickstoff von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.
Schmelzpunkt: 176,92°C

### 20. Hydrochinon/Xenon

Hydrochinon (30 g) wurde in n-Propanol (70 ml) bei 70° C gelöst. Die heiße Lösung wurde in den Hochdruckautoklaven gebracht. Die Lösung wurde mit komprimiertem Xenon von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.

### 21. Hydrochinon/Argon

Hydrochinon (30 g) wurde in n-Propanol (70 ml) bei 70° C gelöst. Die heiße Lösung wurde in den Hochdruckautoklaven gebracht. Die Lösung wurde mit komprimiertem Argon von 300 bar beaufschlagt. Der Hochdruckautoklav wurde 2 h lang auf 80 ° C temperiert. Anschließend wurde die Lösung innerhalb von 5 Tagen auf Raumtemperatur abgekühlt. Die Kristalle wurden abfiltriert und 4 mal mit kaltem n-Propanol (5 ml) gewaschen. Anschließend wurden die Kristalle im Trockenschrank bei 70°C getrocknet.
Schmelzpunkt: 175,67°C

### 22. Harnstoff/Butan

4 g Harnstoff wurden bei 60°C in 12 ml Ethanol gelöst. Die Lösung wurde anschließend in einen Hochdruckautoklaven gebracht und mit einem Butandruck von 150 bar beaufschlagt. Die Lösung wurde innerhalb von 48 H von 60°C auf Raumtemperatur abgekühlt. Die Lösung mit W/G-Kristallen wurde dem Autoklaven entnommen, filtriert und die W/G-Kristalle mit 10 ml kaltem Ethanol gewaschen. Die W/G-Komplexkristalle wurden im Vakuumschrank bei 60°C getrocknet.

### 23. Harnstoff/Isobutan

4 g Harnstoff wurden bei 60°C in 12 ml Ethanol gelöst. Die Lösung wurde anschließend in einen Hochdruckautoklaven gebracht und mit einem Isobutandruck von 150 bar beaufschlagt. Die Lösung wurde innerhalb von 48 h von 60°C auf Raumtemperatur abgekühlt. Die Lösung mit W/G-Kristallen wurde dem Autoklaven entnommen, filtriert und die W/G-Kristalle mit 10 ml kaltem Ethanol gewaschen. Die W/G-Komplexkristalle wurden im Vakuumschrank bei 60°C getrocknet.
Schmelzpunkt: 138,50°C

### 24. Harnstoff/Neopentan

4 g Harnstoff wurden bei 60°C in 12 ml Ethanol gelöst. Die Lösung wurde anschließend in einen Hochdruckautoklaven gebracht und mit einem Neopentandruck von 150 bar beaufschlagt. Die Lösung wurde innerhalb von 48 h von 60°C auf Raumtemperatur abgekühlt. Die Lösung mit W/G-Kristallen wurde dem Autoklaven entnommen, filtriert und die W/G-Kristalle mit 10 ml kaltem Ethanol gewaschen. Die W/G-Komplexkristalle wurden im Vakuumschrank bei 60°C getrocknet.

Schmelzpunkt: 138,79°C

25. Thioharnstoff/Butan

4 g Thioharnstoff wurden bei 60°C in 12 ml Ethanol gelöst. Die Lösung wurde anschließend in einen Hochdruckautoklaven gebracht und mit einem Butandruck von 150 bar beaufschlagt. Die Lösung wurde innerhalb von 60 h auf Raumtemperatur abgekühlt. Die Lösung mit W/G-Kristallen wurde dem Autoklaven entnommen, filtriert und die W/G-Kristalle mit 10 ml kaltem Ethanol gewaschen. Die W/G-Komplexkristalle wurden im Vakuum schrank bei 60°C getrocknet.

26. Thioharnstoff/Isobutan

4 g Thioharnstoff wurden bei 60°C in 20 ml Ethanol gelöst. Die Lösung wurde anschließend in einen Hochdruckautoklaven gebracht und mit einem Isobutandruck von 150 bar beaufschlagt. Die Lösung wurde innerhalb von 60 h auf Raumtemperatur abgekühlt. Die Lösung mit W/G-Kristallen wurde dem Autoklaven entnommen, filtriert und die W/G-Kristalle mit 10 ml kaltem Ethanol gewaschen. Die W/G-Komplexkristalle wurden im Vakuumschrank bei 60°C getrocknet.
Schmelzpunkt: 181,34°C

27. Thioharnstoff/Neopentan

4 g Thioharnstoff wurden bei 60 °C in 20 ml Ethanol gelöst. Die Lösung wurde anschließend in einen Hochdruckautoklaven gebracht und mit einem Neopentandruck von 150 bar beaufschlagt. Die Lösung wurde innerhalb von 60 h auf Raumtemperatur abgekühlt. Die Lösung mit W/G-Kristallen wurde dem Autoklaven entnommen, filtriert und die W/G-Kristalle mit 10 ml kaltem Ethanol gewaschen. Die W/G-Komplexkristalle: wurden im Vakuumschrank bei 60°C getrocknet.

28. Vehikel

A: Für Hydrochinon-, Tri-O-Thymotid-Harnstoff- und Thioharnstoff-W/G-Komplexe eignen sich als Vehikal z.B. folgende Lösungen:
a) 1 % Gelatinelösung
b) 1 % Albuminlösung
c) 10 % Glycerinlösung
d) 15 % Propylenglykollösung
e) Mischungen von Natriumcholat und Phosphatidylcholin in Wasser
f) 0,01 - 1 % Phosphatidylcholindispersion (wäßrig)
g) 1 % Methylcellulose
h) 1 - 2 % Dextranlösung
i) 1 % Agarlösung
j) 2 % Tweenlösung (Tween 80)®
k) 1 % Gummi arabicum
B: Für Dianin-W/G-Komplexe eignen sich z.B. folgende Vehikel:
a) 10 - 20 % 2-(2-methoxyethoxy)-Ethanol
b) Mischungen aus 2-(2methoxyethoxy)-Ethanol (20 %) und
Tween 80® (1 %)

**In-vitro Ultraschalluntersuchungen**

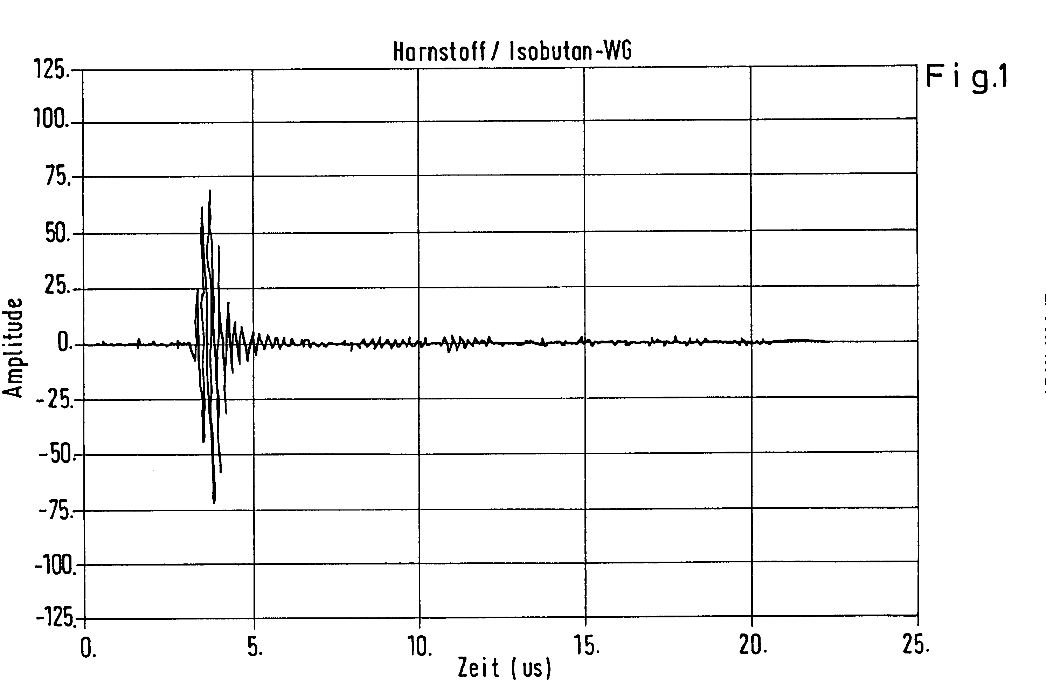

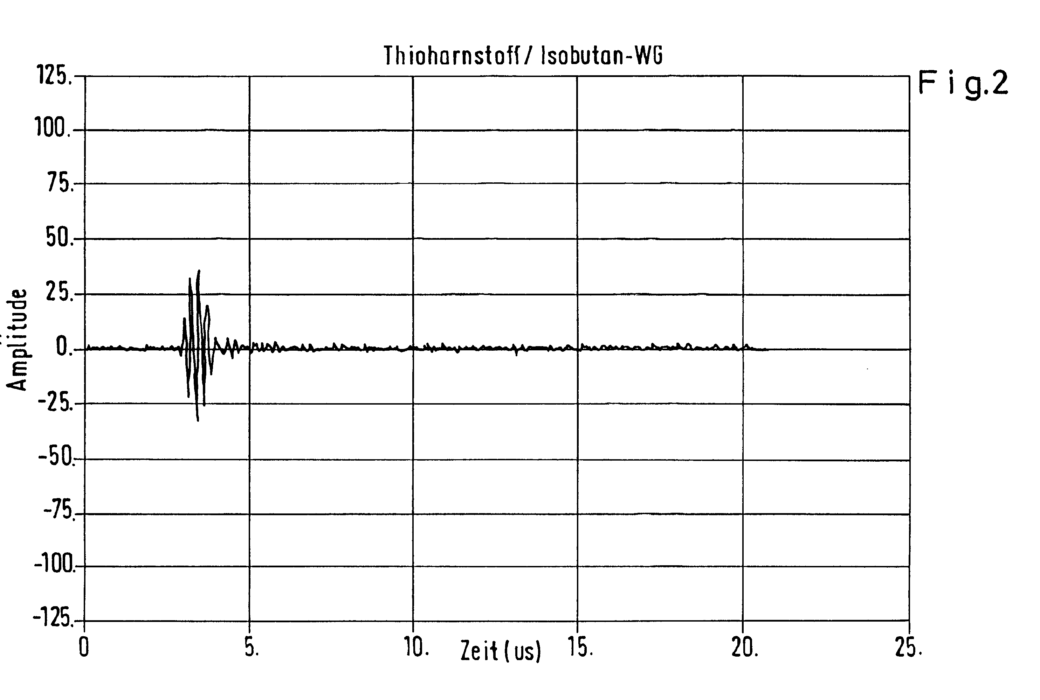

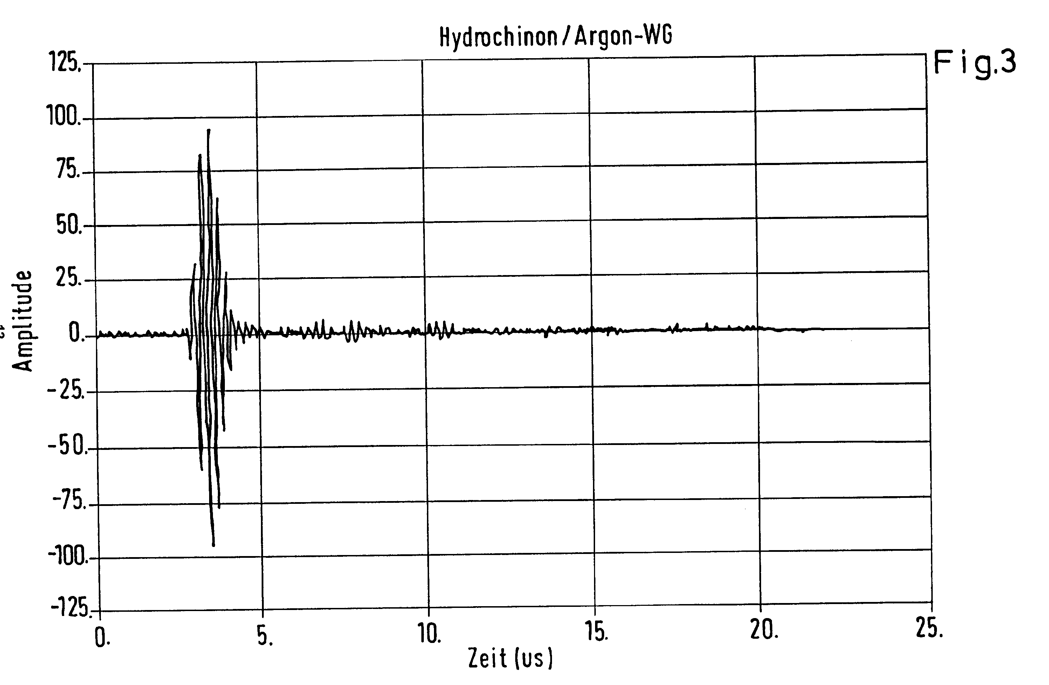

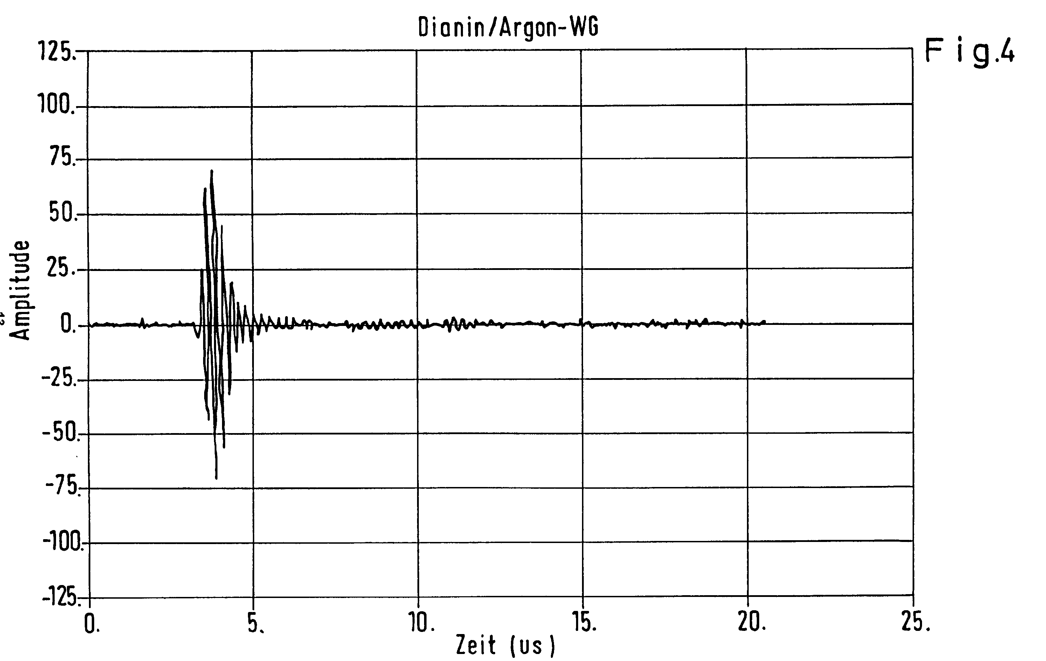

Die akustischen Eigenschaften der W/G-Komplex-Vehikel-Systeme wurden mit in-vitro Ultraschalluntersuchungen bestimmt.
Dazu wurden etwa 1 - 5 mg der W/G-Komplexe in 10 - 20 ml mit einem der genannten Vehikel gemischt und anschließend mit Ultraschall-Scannern untersucht.
Für qualitative Untersuchungen wurder der Ultraschall-Scanner Ekoline 20A/S im Frequenzbereich 1 - 5 MHz verwendet.
Quantitative Messungen der akustischen Eigenschaften wurden in einer Apparatur mit der Ültraschall-Scanner Kraut-Kraemer U.S.I. P-12 bei 4 MHz erhalten. Beispielhaft sind hier Ergebnisse von vier Systemen aufgeführt (Abb. 1 - 4).

Abb. 1: Harnstoff/Isobutan (Beispiel 23) in 2 % Tween 80 Lösung
Abb. 2: Thioharnstoff/Isobutan (Beispiel 26) in 1 % Dextranlösung
Abb. 3: Hydrochinon/Argon (Beispiel 21) in 1 % Gelatinelösung
Abb. 4: Dianin/Argon (Beispiel 11) in 10 % 2-(2-methoxyethoxy)-Ethanol

Zur Erklärung der Ultraschall-Meßapparatur und der daraus gewonnenen Abbildungen:

Die Apparatur besteht aus einem Ultraschall-Sender kombiniert mit einem Empfänger und einer Meßküvette, die die Probe enthält. Zur Messung der akustischen Eigenschaften der Probe wird ein Ultraschall-Impuls ausgesendet. Rückgestreuter Ultraschall wird von dem Empfänger gemessen und durch eine Änderung der Amplitude (siehe Abb.) angezeigt. In den Abbildungen ist jeweils nur eine Amplitudenänderung zu beobachten, die durch Rückstreuung des Ultraschalls an der Vorderwand der Meßküvette resultiert. Eine zweite Amplitudenänderung, die durch Rückstreuung an der Rückwand der Meßküvette resultiert, wird nur mit nichtechogenen Substanzen (z.B. Wasser) erhalten. Im Falle echogener Substanzen erhält man kein zweites Rückstreusignal, da der Ultraschall in der Probe dissipiert bzw. so verändert wird, daß er nicht mehr empfangen werden kann.

## Patentansprüche

1. Mittel bestehend aus Cavitate oder Clathrate-Wirt/Gast-(W/G)-Komplexen, deren Wirtmoleküle sich in einem flüssigen Vehikel unter Freisetzung des Gastes auflösen als Kontrastmittel bei Ultraschall-, Röntgen- oder NMR-Untersuchungen.

2. Mittel nach Anspruch 1 zur Ultraschalluntersuchung enthaltend
als Wirtmoleküle:
Wasser, Harnstoff und dessen Derivate, Thioharnstoff und dessen Derivate, Phenol und substituierte Phenole, Dihydroxybenzole und dessen Derivate, Hydrochinon und substituierte Hydrochinone, Salicylsäure und deren Derivate, Tri-o-Thymotid und dessen Derivate, Ascorbinsäure, Flavine und deren Derivate, Flavanole und deren Derivate, Cyclophane und deren Derivate, Guayacamin, Naphtohydrochinone und deren Derivate, Chromane und deren Derivate, insbesondere 4-p-Hydroxyphenyl-2,2,4-trimethylchroman, 4-p-Hydroxyphenyl-2,2,4-trimethylthiochroman, 4-p-Hydroxyphenyl-2,2,4,7-tetramethylthiochroman, 4-p-Hydroxyphenyl-2,2,4-trimethylselenchroman, Hexahostverbindungen, insbesondere Hexakis(phenylthio)benzol und dessen Derivate, Cyclotriveratrylen und dessen Derivate, 1,1′-Binaphtyl-2,2′-dicarbonsäure und deren Derivate, Oniumverbindungen und deren Derivate, Acetylsalicylsäure, Di-, Tri- und Tetrasalicylide, 9,9′-Spirobifluoren-2,2′-dicarboxylacid, Choleinsäuren, 4-4′Dinitrodiphenyl, Bis-(N,N′-alkylen-benzidin), Bis-(N,N′-tetramethylen-benzidin), Desoxycholsäure, Monoaminonickel(II)-cyanide, Tetra(4-methylpyridin)Nickel(II)-dithiocyanate und deren Derivate, Hexamethylisocyanidoferronchloride, 2-phenyl-3-p(2,2,4-trimethylchroman-4-yl)-phenylquinazolin-4, Cyclotriphosphazone, Tris-1,2-phenyldioxycyclotriphosphazone
als Gastmoleküle:
Edelgase und Edelgasverbindungen, Schwefelhalogenide, Stickstoff und Stickstoffoxide, Kohlenstoffoxide, Wasserstoff und Wasserstoffoxide, Schwefeloxide, Phosphorwasserstoffe, Halogenwasserstoffe, Uranhalogenide und Sauerstoff sowie Kohlenwasserstoffe und deren Derivate, Epoxide, Ether und halogenierte Kohlenwasserstoffe.

## Claims

1. Preparation comprising cavitate or clathrate host/guest (h/g) complexes, the host molecules of which are dissolved in a fluid vehicle to release the guest, as contrast agents in ultrasonic, X-ray or NMR investigations.

2. Preparation according to claim 1 for ultrasonic investigation comprising
as host molecules:
water, urea and derivatives thereof, thiourea and derivatives thereof, phenyl and substituted phenols, dihydroxybenzenes and derivatives thereof, hydroquinone and substituted hydroquinones, salicylic acid and derivatives thereof, tri-o-thymotide and derivatives thereof, ascorbic acid, flavins and derivatives thereof, flavanols and derivatives thereof, cyclophanes and derivatives thereof, guaiacamin, naphthohydroquinones and derivatives thereof, chromans and derivatives thereof, especially 4-p-hydroxyphenyl-2,2,4-trimethylchroman, 4-phydroxyphenyl-2,2,4-trimethylthiochroman, 4-p-hydroxyphenyl-2,2,4,7-tetramethylthiochroman, 4-p-hydroxyphenyl2,2,4-trimethylseleniumchroman, hexahost compounds, especially hexakis(phenylthio)benzene and derivatives thereof, cyclotriveratrylene and derivatives thereof, 1,1′-binaphthyl-2,2′-dicarboxylic acid and derivatives thereof, onium compounds and derivatives thereof, acetylsalicylic acid, di-, tri- and tetra-salicylides, 9,9'-spirobifluorene-2,2'-dicarboxylic acid, choleinic acids, 4,4'-dinitrodiphenyl, bis-(N,N'-alkylene-benzidine), bis-(N,N'-tetramethylene-benzidine), deoxycholic acid, monoaminonickel(II) cyanide, tetra(4-methylpyridine)-nickel(II) dithiocyanates and derivatives thereof, hexamethylisocyanidoferronchlorides, 2-phenyl-3-p(2,2,4-trimethylchroman-4-yl)-phenylquinazoline-4, cyclotriphosphazones, tris-1,2-phenyldioxycyclotriphosphazones and as guest molecules:

inert gases and inert gas compounds, sulphur halides, nitrogen and nitrogen oxides, carbon oxides, hydrogen and hydrogen oxides, sulphur oxides, hydrogen phosphides, hydrogen halides, uranium halides and oxygen as well as hydrocarbons and derivatives thereof, epoxides, ethers and halogenated hydrocarbons.

## Revendications

1. Produits formés de complexes dits logeur/logé (1/1) du genre cavitates ou clathrates, dont les molécules logeuses se dissolvent dans un véhicule liquide en libérant les molécules logées, comme agents de contraste pour des examens par ultrasons, rayons X ou RMN.

2. Produits selon la revendication 1 pour des examens par ultrasons, qui comprennent :

comme molécules logeuses :

eau, urée et ses dérivés, thio-urée et ses dérivés, phénol et dérivés de substitution de celui-ci, dihydroxybenzènes et leurs dérivés, hydroquinone et dérivés de substitution de celle-ci, acide salicylique et ses dérivés, tri-o-thymotide et ses dérivés, acide ascorbique, flavines et leurs dérivés, flavanols et leurs dérivés, cyclophanes et leurs dérivés, guayacamine, naphtohydroquinones et leurs dérivés, cyclodextrine et ses dérivés, en particulier diméthyl-1 cyclodextrine, méthyl-β-cyclodextrine et hydroxypropyl-βcyclodextrine, chromanes et leurs dérivés, notamment 4-p-hydroxyphényl-2,2,4-triméthylchromane, 4-p-hydroxyphényl2,2,4-triméthylthiochromane, 4-p-hydroxyphényl-2,2,4,7-tétraméthylthiochromane et 4-p-hydroxyphényl-2,2,4-triméthylsélénochromane, composés hexavalents, notamment l'hexakis(phénylthio)benzène et ses dérivés, cyclotrivératrylène et ses dérivés, acide 1,1'-binaphtyl2,2'-dicarboxylique et ses dérivés, composés d'onium et leurs dérivés, acide acétylsalicylique, di-, tri- et tétrasalicylides, acide 9,9'-sprirobifluorène-2,2'-dicarboxylique, acides choliques, 4,4'-dinitrobiphényle, bis-(N,N'-alkylène-benzidines), bis-(N,N'-tétraméthylènebenzidine), acide désoxycholique, cyanures de monoaminonickel divalent, dithiocyanates de tétra-(4-méthyl-pyridine)-nickel divalent et leurs dérivés, chlorures d'hexaméthylisocyanidoferrones, 2-phényl-3-p-(2,2,4-triméthylchromane-4-yl)-phénylquinazoline-4, cyclotriphosphaszones et tris-1,2-phényldioxycyclotriphosphazones,

et comme molécules logées :

gaz rares et composés de gaz rares, halogénures de soufre, azote et oxydes d'azote, oxydes de carbone, hydrogène et oxydes d'hydrogène, oxydes de soufre, phosphures et halogénures d'hydrogène, halogénures d'uranes et oxygène, ainsi que des hydrocarbures et leurs dérivés, époxydes, éthers et hydrocarbures halogénés.

Harnstoff / Isobutan-WG

Fig.1

EP 0 357 163 B1

Thioharnstoff/ Isobutan-WG

Fig.2

EP 0 357 163 B1

Hydrochinon/Argon-WG

Fig.3

EP 0 357 163 B1

Dianin/Argon-WG

Fig.4

EP 0 357 163 B1